# EUROPEAN PATENT SPECIFICATION

(11) **EP 3 482 674 B1**
(45) Date of publication and mention of the grant of the patent: **30.03.2022**
(21) Application number: 17200972.2
(22) Date of filing: 10.11.2017
(51) Int. Cl.: A61B 1/06, A61B 1/00, A61B 3/06, A61B 3/00

(54) **SYSTEM COMPRISING AN OPTICAL INSTRUMENT**
SYSTEM MIT EINEM OPTISCHEN INSTRUMENT
SYSTÈME COMPRENANT UN INSTRUMENT OPTIQUE

(43) Date of publication of application: 15.05.2019
(73) Proprietor: Maxer Endoscopy GmbH, 78573 Wurmlingen (DE)
(72) Inventor: JOSHI, Shirish Raghunath, 78549 Spaichingen (DE)
(74) Representative: Patentanwälte und Rechtsanwalt Weiß, Arat & Partner mbB

(56) References cited:
- EP-A1- 1 099 405
- WO-A1-2012/154279
- WO-A1-2015/199806
- CN-A- 102 938 247
- US-A1- 2015 070 773
- US-A1- 2016 180 780
- US-A1- 2017 039 958
- US-A1- 2017 181 619

## Description

### Technical Area

The present invention is directed at a system comprising an optical instrument for use in the field of surgery.

### State of the Art

Systems comprising an optical instrument for use in the field of medicine and methods for operating such systems are widely known.

For example, endoscopic systems are known. Endoscopic systems are widely used in many areas of medicine and also in non-medical fields to investigate cavities which are difficult to access.

Rigid and flexible endoscopes are known which may contain a plurality of lenses, a plurality of rod lenses or light guiding cables such as fibre optical cables in order to order to allow the user to investigate cavities and the like which would be inaccessible otherwise.

Moreover, video endoscopes are known which comprise an image sensor and an exit region of a fibre optical cable or the like at the distal end which can be introduced into the body of a patient. Light from a light source is introduced into the fibre optical cable and leaves the latter at the exit region to illuminate the area or organ to be investigated. The image sensor acquires the desired image. The image data acquired by the image sensor are transported either by respective cables within the endoscope or wireless to a processing or control unit and are shown on a suitable display or screen.

In various situations ambient light, i.e. for example the light being present in the operating room, causes problems and especially leads to interference with the optimal use of the endoscope or the endoscopic system. This light may be sunlight or artificial illumination from a ceiling light or an operating light, for example.

Known systems comprising optical instruments such as endoscopic systems are used, for example, in different operating rooms or both, in the doctor's office and in the operating room. Light conditions vary broadly between these rooms and offices but also within the same room or office resulting from different combinations of ceiling lights, for example, illuminating this room and/or from changing sunlight entering the room through a window.

If the brightness of the image seen through an ocular lens or especially on a display or screen such as a flat panel display or the like is too high, the eyes of the user, e.g. the surgeon, fatigue more rapidly and the resolution and detail of the image is lost.

If the image brightness is too low, the user experiences difficulties in trying to see details in the darker areas of the image. This problem aggravates proportional to the intensity of incident ambient light.

Known systems comprising optical instruments such as endoscopic systems are pre-adjusted to a particular brightness level which can thereafter only be changed manually with great effort. However, during a surgery, for example, the surgeon should concentrate only on the patient without adjusting brightness of the endoscopic system.

Systems are known which deal with ambient-light-problems.

Document DE 10 2012 100 955 A1 discloses a system which may be used in endoscopy. For handling problems caused by ambient light, DE 10 2012 100 955 A1 suggests to use physical or digital filters which only allow light of a desired wavelength range to reach the image sensor.

However, using filters is impossible in many applications. For example, when both, the ambient light and the remitted or emitted light to be observed lie in the same wavelength range, filters cannot be used to overcome ambient-light-problems because such filters can only discriminate light with respect to wavelength but not with respect to its origin, i.e. either ambient light and light produced by the endoscopic system's light source.

Document WO 2012 054 268 A2 discloses a multifunctional medical device for telemedicine applications, wherein the medical device may be an endoscope. The medical device may comprise a control unit which in turn may comprise an ambient light sensor which is connected to a processing unit.

Document WO 1994 015 521 A1 and document WO 1994 028 783 A1 disclose a medical video endoscope system with a first and a second image sensor and a camera controller to control an electrical shutter. Ambient light changes which may lead to brightness variations are compensated for by the combination of the electrical shutter and a mechanical shutter. However, ambient light conditions are registered by the two sensors which are also used for acquiring the video image for the endoscopic application.

Document EP 1 099 405 A1 discloses an endoscope which has a CCD incorporated in the distal part of an insertion unit thereof.

Documents US 2017 0 181 619 A1 and WO 2012 154 279 A1 disclose vision testing systems.

Document US 2015 0 070 773 A1 discloses a virtual image display apparatus.

In particular, said document describes a system comprising an optical instrument for use in the field of surgery wherein the system comprises a radiation source and a screen for providing information to a user, wherein the optical instrument comprises an image sensor for acquiring a desired image, wherein said system is configured to adjust a radiation intensity of the radiation source and/or a brightness of the screen.

### Problem to be solved

The present invention seeks to provide a system as mentioned above which overcomes the above-mentioned drawbacks

### Solution of the Problem

A system according to the present invention, this system comprising an optical instrument for use in the field of surgery comprises the steps of registering ambient radiation of a pre-defined wavelength range and adjusting a radiation intensity of the radiation leaving the optical instrument and/or adjusting the brightness of the screen in response to the ambient radiation registered by an ambient radiation sensor. The system also comprises a radiation source and a screen for providing information to a user such as a surgeon. Usually this information mainly consists of the image registered by the optical instrument.

When referring to radiation or ambient radiation, especially the radiation registered by the sensor, preferably an intensity of that (ambient) radiation is meant herein.

Preferably, when a high intensity of ambient radiation is registered, the adjustment is done by increasing the radiation intensity of the radiation leaving the optical instrument and/or by increasing brightness of the screen. Correspondingly, when a low intensity of ambient radiation is registered, the adjustment is done by decreasing the radiation intensity of the radiation leaving the optical instrument and/or by decreasing brightness of the screen.

The ambient radiation sensor may be any suitable sensor, for example a photodetector using the photoelectric effect.

In the context of the present invention, radiation may comprise visible light or infrared radiation. Also ultraviolet radiation may be comprised.

Visible light in the context of this invention is electromagnetic radiation within the wavelength range between 380 nm and 780 nm or between 400 nm and 700 nm.

Infrared radiation in the context of this invention is electromagnetic radiation within the wavelength range between 780 nm and 1 mm (1.000 µm) or between 700 nm and 1 mm (1.000 µm).

Near infrared radiation (NIR) in the context of this invention is electromagnetic radiation within the wavelength range between 780 nm and 1400 nm or between 700 nm and 1400 nm.

A screen according to the present invention may be a flat panel display. Even more preferably the present invention is directed at endoscopic systems, especially at video endoscopes and video endoscopic systems.

The system preferably also comprises means for guiding the radiation of the radiation source to the area under investigation. Such means are present in known endoscopes.

The radiation intensity of the radiation leaving the optical instrument may be adjusted in a variety of ways. For example, the light source (or radiation source with respect to infrared radiation, for example) can be down-regulated or suitable automatically driven filters can be used.

The brightness of the screen can be adjusted in the way which is known from flat panel displays used with common personal computers and the like.

By adjusting the radiation intensity or the brightness of the screen as described above, the problems known from the state of the art can be overcome since the image brightness is optimized automatically and thus never too high or too low.

The advantages provided by the method described above are readily evident with respect to visible light applications, i.e. when the pre-defined wavelength range consists of at least a subrange of the visible light spectrum.

However, there are other advantages with respect to applications using infrared radiation, for example. When employing infrared radiation during surgery by using techniques such as NIR-fluorescence or thermography which are discussed in more detail below, the pre-defined wavelength range may consist of at least a subrange of the infrared range, e.g. the near infrared radiation (NIR) range. Thus, for example, the radiation intensity of the radiation leaving the optical instrument may be adjusted in response to the ambient infrared radiation. In this way interference of ambient infrared radiation is avoided and only remitted or fluorescent radiation which is to be investigated is detected by the optical instrument.

Preferably, said registering function and/or that said adjusting function can be disabled. Disabling one or both of the aforementioned functions is preferably possible at any given time, for example, before a surgery begins or even during the surgery.

There are rare situations in which a user, e.g. a surgeon, has to increase or decrease ambient radiation, especially artificial illumination such as ceiling lights or operating lights, to the maximal extent and accept aggravated image quality. In some of these situations the automatic adjustment of the radiation intensity and/or screen brightness described above is not advantageous. In order to allow for such deliberate minimization or maximization of ambient radiation without automatic adjustments conducted by the system, the disabling function described above may be provided.

Preferably, a feedback on the ambient radiation conditions is given to a user.

The feedback may be provided by any part of the system.

The feedback may be provided immediately or shortly after starting or booting the system. Thus, when starting an endoscopic system prior to the surgery, for example, an immediate feedback on the ambient radiation conditions is given to the user. This may be advantageous for the user since the system according to the present invention thus allow for testing the ambient radiation in the room by starting or booting the system. Thus, undesirable ambient radiation conditions may be changed before the surgery begins, since the system is usually booted some time prior to beginning the surgery.

However, ambient radiation conditions may change over time, e.g. because incident sunlight varies over time or simply because light sources of artificial illumination such as ceiling lights or operating lights are switched off or additional light sources are switched on during surgery. Therefore, preferably, the feedback function described herein is active all the time and checks for ambient light conditions as described below.

The feedback can be, for example, a visual feedback or an acoustic feedback. The visual feedback may be given on the flat panel display, e.g. by appropriate pictograms, numbers or the like.

The feedback may be generated by comparing the radiation intensity registered by the radiation sensor with radiation intensity values stored in the system or with radiation values registered during former surgeries or with radiation values pre-defined by the user or a combination of the aforementioned values. Suitably, at least a minimum and a maximum intensity of a preferred radiation intensity range are stored in the system.

After comparing the values the system may for example indicate the deviation of the registered radiation intensity from the values stored in the system. This may be done by giving the deviation in percent or by providing the radiation intensity in a suitable unit together with the registered information whether that radiation is in the optimal range or too low or too high. In short, for example relative or absolute radiation values may be given which may also be translated into qualitative statements which can readily be interpreted by the user.

Additionally or alternatively the feedback may include instructions and guidance to the user by recommending increasing the ambient radiation in case the radiation intensity measured by the sensor is lower than a lower border of the preferred or optimal range. Accordingly, the instructions to reduce the ambient radiation may be given when the radiation intensity measured by the radiation sensor is above the upper border of the optimal or preferred intensity range.

The system according to the present invention comprises automatic adjustment of radiation intensity leaving the optical instrument and/or automatic adjustment of the brightness of the screen. However, in some situations the ambient radiation, especially ambient (visible) light may be extremely high or extremely low resulting in suboptimal overall light conditions. Therefore, upper and/or lower borders for the adjustment function may be included or implemented. Thus, the adjustment is only conducted within these borders and, for example, an excess of illumination in the operating room does not lead to the screen brightness to increase to values which even worsen the recognisability of the image.

Thus, also extremely unfavourable light conditions such as complete darkness do not result in the radiation intensity to drop to zero or near zero and/or to turn the screen dark. Such borders are of advantage in the rare occasions where such extremely unfavourable radiation conditions are accepted or even chosen deliberately for a specific reason. Such borders are also of advantage in occasions where such unfavourable conditions occur by chance or accident and respective adjustments would even worsen the situation, e.g. when turning the screen dark when the operating light turns dark.

On the one hand, as described above, there may be an upper and a lower border for the radiation intensity leaving the optical instrument and/or for the brightness of the screen. These borders prevent the system from conducting an adjustment which would even worsen the overall situation for the user, e.g. for the surgeon.

On the other hand, alternatively or additionally to the aforementioned borders, the feedback function described above is also advantageous in situations with highly undesirable ambient radiation conditions. Especially in combination, both functions are highly advantageous, since the adjustment is only conducted within acceptable borders without worsening the situation and at the same time the feedback is given instructing the user to improve the ambient radiation conditions. This guarantees optimal ambient radiation conditions all the time during surgery.

In short, there may be a range of ambient radiation where automatic adjustment is suitable while outside of that range adjustment even worsens the situation. This problem may be overcome by the method described in detail above, which comprises automatic adjustment within a pre-defined range of ambient radiation and which provides a feedback to the user when ambient radiation should be manually changed.

The aforementioned steps of course apply to visible light or visible radiation.

Moreover, the aforementioned steps also apply to infrared radiation, which is often used in surgery. In visible light applications the user, for example the surgeon, may at least partially judge ambient light intensity by himself and is supported by the adjustment function and/or by the feedback as described above. However, in infrared radiation applications the user or surgeon cannot judge ambient infrared radiation with his eyes. Thus, if the desired image cannot be obtained the user cannot even guess whether there is a technical problem or whether the system works properly and ambient infrared illumination is the problem.

Problems arise, for example, when remitted or fluorescent infrared radiation in the wavelength range of interest is present within ambient radiation. For example, especially near infrared radiation (NIR) constitutes a significant proportion of sunlight. Also many sources of artificial illumination generate a significant proportion of infrared radiation.

In the field of surgery, active and especially passive infrared thermography and NIR fluorescence are widely used. These processes are widely known.

When conducting minimal invasive surgery and using NIR fluorescence techniques, ambient near infrared radiation may be registered by the optical instrument because that radiation may in some way reach the area under investigation. Moreover, sometimes NIR techniques are used in open surgery, where, when present, ambient infrared radiation most certainly reaches the area under investigation.

In these situations the aforementioned steps provide a significant improvement to the surgeon. When, for example, the image sensor of the optical instrument such as a video endoscope is configured to register near infrared radiation, according to an embodiment of the present invention the system is configured so that the ambient radiation sensor automatically registers radiation in the near infrared wavelength range in order to detect interfering radiation. Thus, interference by ambient infrared radiation can be avoided.

Of course, this example may be transferred to any other wavelength range, i.e. the ambient radiation sensor may always be configured to detect ambient radiation in the wavelength range which is being investigated by the user with the help of the optical instrument.

When high ambient infrared radiation is detected, the radiation intensity of infrared radiation leaving the optical instrument may be adjusted, i.e. increased, accordingly.

Additionally, when ambient infrared radiation exceeds a pre-defined border, a feedback may be given to the user to reduce infrared ambient radiation which may be accomplished by reducing or even switching out artificial illumination, for example.

As described above, also with respect to infrared radiation there may be an optimal range within which, for example, automatic adjustment of the radiation intensity leaving the optical instrument is desirable; whereas outside of which providing a feedback to the user in order to change ambient infrared radiation conditions is more favourable.

Thus, as described in detail above, the pre-defined wavelength range may consist of infrared radiation.

Of course, alternative embodiments are encompassed by the present invention.

For example, the system may comprise two or more ambient radiation sensors. For example, a first ambient radiation sensor may register infrared radiation or NIR, while a second ambient radiation sensor may register visible light radiation. Such an embodiment comprising at least a first and a second ambient radiation sensor is advantageous when the system is used for both, infrared and visible light applications. Such an embodiment is especially advantageous when overlay-images consisting of the visible light image and the infrared image are to be provided.

The sensor may also be suitable for detecting both infrared and visible light radiation. The system may then split or divide the registered infrared radiation intensity and the registered visible light intensity, for example with digital filters or with prisms and physical filters or the like. Thus, the functions described above may be provided for both, ambient infrared radiation and ambient visible light by using only one ambient radiation sensor.

In general, at least a first and a second pre-defined wavelength range may be registered by at least one ambient radiation sensor.

Regardless of whether visible and infrared radiation is registered by one or two sensors, the system may comprise more than one radiation source. In case the system comprises an infrared radiation source and a visible light source, both radiation sources may be adjusted individually based on the registered radiation, i.e. the registered ambient visible light only leads to adjustment of the radiation intensity of the visible light source.

The method may comprise additional features.

For example, a test mode may be provided for the ambient radiation sensor. This test mode may at least comprise registering the radiation intensity of the pre-defined wavelength range without influence or manipulation of the user and registering the radiation intensity of the pre-defined wavelength when the user directs a part which emits radiation such as the distal end of the endoscope at the ambient radiation sensor. Thus, a fast and easy test can be conducted by guiding the user through the aforementioned steps and by comparing the registered intensity values with stored values reflecting acceptable intensity ranges for the chosen wavelength range in the at least two test situations, i.e. without manipulation by the user and with the user directing the endoscope at the sensor.

Although most functions and features were described with respect to endoscopes and endoscopic systems, these functions and features may also be included for operating other systems and other optical instruments.

The present invention is directed at a system which is operated as described above and which is highly useful in surgery.

A system according to the present invention comprises an optical instrument for use in the field of surgery. The system comprises an ambient radiation sensor, a radiation source and a screen for providing information to a user. The ambient radiation sensor is configured to register ambient radiation of a pre-defined wavelength range. The system is configured to adjust a radiation intensity of the radiation source and/or a brightness of the screen in response to the registered ambient radiation.

The system may be an endoscopic system. Such systems comprise the endoscope, which may be connected to a control unit comprising the light source or radiation source. The radiation source or light source may also be a separate part which is not integrated in the control unit. Alternatively, the radiation source may also be integrated in the optical instrument, for example in the endoscope. The screen may be part of the control unit or it may be a separate part connected to that control unit. The ambient radiation sensor may be mounted in a housing of the control unit. The ambient radiation sensor may also be mounted elsewhere, for example on the endoscope itself at a place which is not introduced into the body of the patient and which is not covered by the hands of the surgeon during surgery.

Such a system is suitable for use in the medical field. However, such a system may also be used in a variety of other technical fields.

The system may be configured to give a feedback on the ambient radiation conditions. This feedback function is explained in more detail above with respect to the method described above.

The ambient radiation sensor may be configured to register ambient radiation of a pre-defined wavelength range of the infrared part of the spectrum.

The optical instrument comprises an image sensor. Thus, in a preferred example, the system comprises an optical instrument which comprises an image sensor and the system additionally comprises an ambient radiation sensor.

The optical instrument may be an endoscope.

According to an embodiment of the present invention an endoscopic system is provided comprising an endoscope with an image sensor, a control unit and a radiation source. Such a system is known and commercially available. According to the present invention, the system is equipped with at least a first ambient radiation sensor which works as described in detail above. This ambient radiation sensor may be included in the housing of the control unit which may also comprise the radiation source.

For example, a given endoscopic system may, according to the present invention, be equipped with an ambient radiation sensor which was described in detail above. Such a given endoscopic system may comprise an optical instrument for use in the field of surgery comprising a radiation source and a screen.

## Claims

1. System comprising an optical instrument for use in the field of surgery wherein the system comprises an ambient radiation sensor, a radiation source and a screen for providing information to a user,
wherein the optical instrument comprises an image sensor for acquiring a desired image,
wherein said ambient radiation sensor is configured to register ambient radiation of a pre-defined wavelength range,
wherein said system is configured to adjust a radiation intensity of the radiation source and/or a brightness of the screen in response to the registered ambient radiation.

2. System according to claim 1, **characterized in that** the system comprises a means configured to disable said ambient radiation sensor and/or said adjusting function.

3. System according to at least one of the claims 1 and 2, **characterized in that** the system is configured to give a feedback on the ambient radiation conditions to a user.

4. System according to at least one of the claims 1 to 3, **characterized in that** said ambient radiation sensor is configured to register ambient radiation of a pre-defined wavelength range of the infrared part of the spectrum.

5. System according to at least one of the claims 1 to 4, **characterized in that** the optical instrument is an endoscope.

## Patentansprüche

1. System umfassend ein optisches Instrument zur Verwendung auf dem Gebiet der Chirurgie, wobei das System einen Umgebungsstrahlungssensor, eine Strahlungsquelle und einen Bildschirm zur Bereitstellung von Informationen für einen Benutzer umfasst,
wobei das optische Instrument einen Bildsensor zur Erfassung eines gewünschten Bildes umfasst,
wobei der Umgebungsstrahlungssensor so konfiguriert ist, dass er Umgebungsstrahlung in einem vordefinierten Wellenlängenbereich registriert,
wobei das genannte System so konfiguriert ist, dass es eine Strahlungsintensität der Strahlungsquelle und/oder eine Helligkeit des Bildschirms als Reaktion auf die registrierte Umgebungsstrahlung einstellt.

2. System nach Anspruch 1, **dadurch gekennzeichnet, dass** das System ein Element umfasst, das so konfiguriert ist, dass der Umgebungsstrahlungssensor und/oder die Einstellfunktion deaktiviert werden kann.

3. System nach mindestens einem der Ansprüche 1 und 2, **dadurch gekennzeichnet, dass** das System so konfiguriert ist, dass es einem Benutzer eine Rückmeldung über die Umgebungsstrahlungsbedingungen gibt.

4. System nach mindestens einem der Ansprüche 1 bis 3, **dadurch gekennzeichnet, dass** der Umgebungsstrahlungssensor so konfiguriert ist, dass er Umgebungsstrahlung eines vordefinierten Wellenlängenbereichs des infraroten Teils des Spektrums erfasst.

5. System nach mindestens einem der Ansprüche 1 bis 4, **dadurch gekennzeichnet, dass** das optische Instrument ein Endoskop ist.

## Revendications

1. Système comprenant un instrument optique destiné à être utilisé dans le domaine de la chirurgie, le système comprenant un capteur de rayonnement ambiant, une source de rayonnement et un écran servant à fournir des informations à un utilisateur,
l'instrument optique comprenant un capteur d'image servant à acquérir une image souhaitée, ledit capteur de rayonnement ambiant étant configuré pour enregistrer le rayonnement ambiant d'une plage de longueurs d'onde prédéfinie,
ledit système étant configuré pour régler une intensité de rayonnement de la source de rayonnement et/ou une luminosité de l'écran en réponse au rayonnement ambiant enregistré.

2. Système selon la revendication 1, **caractérisé en ce que** le système comprend un moyen configuré pour désactiver ledit capteur de rayonnement ambiant et/ou ladite fonction de réglage.

3. Système selon au moins l'une des revendications 1 et 2, **caractérisé en ce que** le système est configuré pour fournir un retour d'informations sur les conditions de rayonnement ambiant à un utilisateur.

4. Système selon au moins l'une des revendications 1 à 3, **caractérisé en ce que** ledit capteur de rayonnement ambiant est configuré pour enregistrer le rayonnement ambiant d'une plage de longueurs d'onde prédéfinie de la partie infrarouge du spectre.

5. Système selon au moins l'une des revendications 1 à 4, **caractérisé en ce que** l'instrument optique est un endoscope.
